# EUROPEAN PATENT APPLICATION

(11) **EP 3 878 512 A1**
(43) Date of publication of application: **15.09.2021**
(21) Application number: 19881001.2
(22) Date of filing: 07.11.2019
(51) Int. Cl.: A61P 3/10, A61P 43/00, A61F 2/28, A61K 9/00, A61K 35/12, A61K 35/39, A61K 47/36, A61K 47/42

(54) **TRANSPLANT AND USE THEREOF**

(30) Priority: 09.11.2018 JP 2018211288
(71) Applicant: The University of Tokyo, Bunkyo-ku, Tokyo 113-8654 (JP)
(72) Inventor: TAKEUCHI Shoji, Tokyo 113-8654 (JP); OZAWA Fumisato, Tokyo 113-8654 (JP); NAGATA Shogo, Tokyo 113-8654 (JP)
(74) Representative: Ernest Gutmann - Yves Plasseraud S.A.S.
(86) International application number: PCT/JP2019/043582
(87) International publication number: WO 2020/095974

(57) **Abstract**

A graft is provided having a shell part that comes into contact with a body fluid when the graft is transplanted into a living body; and a core part that is located inside the shell part and does not substantially come into contact with the body fluid, in which the core part contains a plurality of first hydrogel fibers containing cells, the shell part contains a second hydrogel functioning as a semipermeable membrane, and the graft has a size with an outer diameter of greater than 1.5 mm.

## Description

### Technical Field

The present invention relates to a graft and use thereof. More particularly, the present invention relates to a graft, a graft production device, and a method for producing a graft. Priority is claimed on Japanese Patent Application No. 2018-211288, filed in Japan on November 9, 2018, the content of which is incorporated herein by reference.

### Background Art

In recent years, there has been a demand for technologies for safely transplanting cells differentiated from pluripotent stem cells such as induced pluripotent stem cells (iPS cells) and embryonic stem cells (ES cells), cells differentiated from tissue stem cells, cells collected from human beings and pigs, and the like into living bodies.

Usually, when allogeneic or xenogeneic cells are transplanted into a living body, the transplanted cells are rejected by the immune system of the host, and the functions of the transplanted cells deteriorate. Thus, in order to solve this problem, a method of transplanting encapsulated cells as a graft has been examined (see, for example, Patent Document 1).

### Citation List

### Patent Document

### [Patent Document 1]

Published Japanese Translation No. 2006-503080 of the PCT International Publication

### Summary of Invention

### Technical Problem

However, in the method described in Patent Document 1, it may be difficult to take out a graft in an emergency or the like, and there is still a problem regarding the functional deterioration of the graft caused by a foreign body reaction. Furthermore, in a case where a large number of cells are transplanted, the graft tends to be large, and the number of functional transplanted cells may decrease due to central cell necrosis or the like.

Thus, an object of the present invention is to provide a graft with which cells can be transplanted into a living body without being subjected to an immune attack and can be taken out of the living body.

### Solution to Problem

The present invention includes the following embodiments.
[1] A graft having a shell part that comes into contact with a body fluid when the graft is transplanted into a living body; and a core part that is located inside the shell part and does not substantially come into contact with the body fluid, in which the core part contains a plurality of first hydrogel fibers containing cells, the shell part contains a second hydrogel that functions as a semipermeable membrane, and the graft has a size with an outer diameter of 1 mm or greater.
[2] The graft according to [1], wherein the semipermeable membrane has a molecular weight cut-off of 70,000 to 500,000.
[3] The graft according to [1] or [2], wherein the graft has a recess into which the body fluid intrudes when the graft is transplanted into the living body.
[4] The graft according to any one of [1] to [3], wherein two weeks after being transplanted into the living body, the number of cells derived from the living body and adhering to a surface is 5×10⁵ cells/cm² or less.
[5] The graft according to any one of [1] to [4], wherein cells contained in the first hydrogel fibers are allogeneic or xenogeneic to the living body.
[6] The graft according to any one of [1] to [5], wherein a distance between a surface of the graft and the cells contained in the first hydrogel fibers is a distance over which the cells are viable.
[7] The graft according to any one of [1] to [6], wherein the cells contained in the first hydrogel fibers are cells of at least one selected from the group consisting of endocrine cells, exocrine cells, neural cells, skeletal muscle cells, blood cells, and interstitial cells.
[8] The graft according to any one of [1] to [7], wherein the cells contained in the first hydrogel fibers include a plurality of kinds of cells.
[9] A graft production device including a container part and a plurality of columnar jigs that are attachably and detachably disposed inside the container part.
[10] A method for producing the graft according to any one of [1] to [8], the method including: a step of injecting a second sol into the container part of the graft production device according to [9] and forming a second hydrogel; a step of removing the plurality of columnar jigs after the formation of the second hydrogel and obtaining a second hydrogel in which the portions where the columnar jigs have existed form recesses; and a step of injecting a first sol containing cells into the recesses and forming a first hydrogel.
[11] A graft production device including a first cylindrical part and a plurality of second cylindrical parts disposed inside the first cylindrical part.
[12] A method for producing the graft according to any one of [1] to [8], the method including a step of supplying a second sol through one side of the graft production device according to [11] into the first cylindrical part, and forming a laminar flow of the second sol that is discharged through the other side of the graft production device while gelling the second sol to form a second hydrogel; and a step of supplying a first sol containing cells through one side of the graft production device into the second cylindrical parts, and forming a laminar flow of the first sol that is discharged through the other side of the graft production device while gelling the first sol to form a first hydrogel, the two steps being carried out simultaneously.

### Advantageous Effects of Invention

According to the present invention, a graft can be provided with which cells can be transplanted into a living body without being subjected to an immune attack and can also be taken out of the living body.

### Brief Description of Drawings

Fig. 1(a) is a schematic diagram showing an example of a graft. Fig. 1(b) is a photograph of a graft having the structure shown in Fig. 1 (a).
Fig. 2(a) is a perspective view showing the structure of an example of a modification example of the graft. Fig. 2(b) is a cross-sectional view showing the structure of an example of a modification example of the graft.
Fig. 3 is a schematic diagram showing the structure of an example of a graft production device.
Fig. 4(a) is a schematic diagram showing the structure of an example of the graft production device. Fig. 4(b) is a photograph showing a specific example of the graft production device.
Fig. 5 is a schematic diagram showing an example of a production process for a hydrogel fiber.
Fig. 6 is a graph showing the results of Experimental Example 2.
Fig. 7 is a photograph showing a state in which a graft is being transplanted into a mouse in Experimental Example 4.
Fig. 8 is a graph showing the results of measuring a transition in the blood glucose concentration in mice in Experimental Example 4.
Fig. 9 is a graph showing the results of measuring the concentration of human C-peptide in the blood of mice in Experimental Example 4.
Fig. 10 is a graph showing the results of measuring a transition in the blood glucose concentration in mice in Experimental Example 5.
Fig. 11 is a graph showing the results of measuring the concentration of human C-peptide in the blood of mice in Experimental Example 5.
Fig. 12 is a photograph of a graft extracted in Experimental Example 6.
Fig. 13 is a graph showing the measurement results for the concentrations of C-peptide and glucagon in Experimental Example 6.
Figs. 14(a) and 14(b) are microphotographs showing the results of hematoxylin and eosin staining of a thin sliced section of a graft in Experimental Example 6.

### Description of Embodiments

Hereinafter, embodiments of the present invention will be described in detail with reference to the drawings according to circumstances. In the drawings, the same or equivalent portions will be denoted by the same or corresponding reference numerals, and any overlapping description will not be repeated. The dimensional ratio in each figure has some portions exaggerated for explanation and does not necessarily match the actual dimensional ratio.

### [Graft]

According to an embodiment, the present invention provides a graft having a shell part that comes into contact with a body fluid when the graft is transplanted into a living body; and a core part that is located inside the shell part and does not substantially come into contact with the body fluid, in which the core part includes a plurality of first hydrogel fibers containing cells, the shell part contains a second hydrogel functioning as a semipermeable membrane, and the graft has a size with an outer diameter of 1 mm or greater.

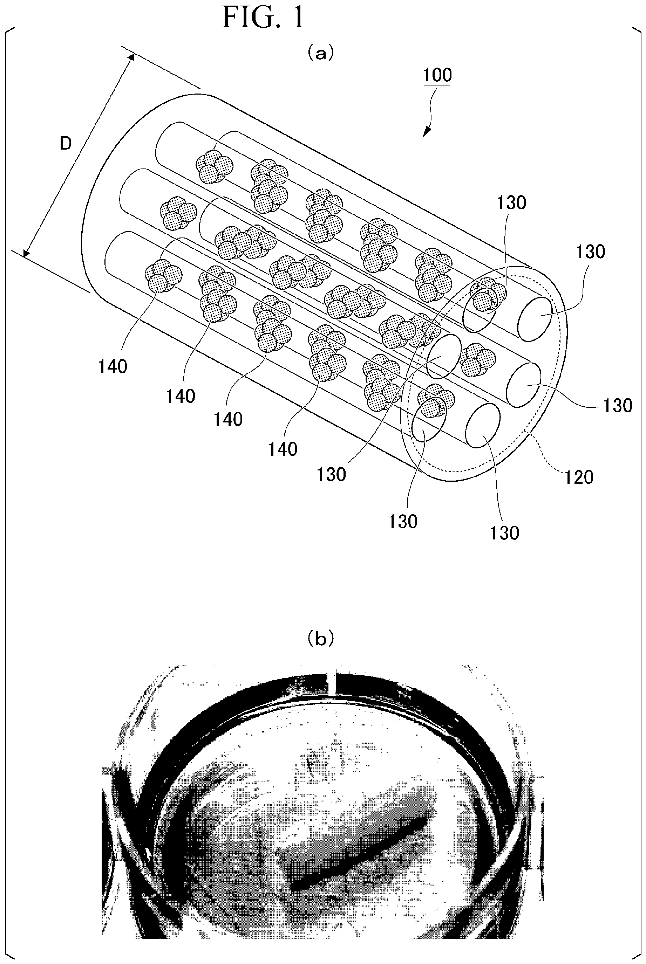

Fig. 1(a) is a schematic diagram showing an example of a graft of the present embodiment. Fig. 1(b) is a photograph of a graft having the structure shown in Fig. 1(a). As shown in Fig. 1(a), the graft 100 has a shell part 110 and a core part 120. The shell part 110 is located on the surface of the graft and comes into contact with a body fluid when the graft is transplanted into a living body. The core part 120 is located inside the shell part 110 and does not substantially come into contact with a body fluid even when the graft is transplanted into a living body. Here, when it is said to be substantially not in contact with a body fluid, it means that, for example, at the edges of the graft, the core part may be exposed at the surface of the graft, and in this portion, the core part is allowed to come into contact with a body fluid.

The core part 120 includes a plurality of first hydrogel fibers 130. A hydrogel is a gel having a three-dimensional network structure containing a large amount of water. A hydrogel can be formed by gelling a sol of an aqueous solution of a water-soluble polymer or the like, and the details will be described later. According to the present specification, a hydrogel fiber means a hydrogel in a fibrous form.

As the core part 120 includes a plurality of first hydrogel fibers 130, the number of cells 140 contained in the graft can be increased. Furthermore, the outer diameter D, which will be described later, is adjusted to an appropriate size, and the distance between the surface of the graft 100 and the cells 140 can be easily adjusted to a distance over which the cells 140 are viable.

The hydrogel fibers 130 contain cells 140. The shell part 110 is composed of a second hydrogel that functions as a semipermeable membrane. It is preferable that the shell part 110 function as a semipermeable membrane having a molecular weight cut-off of 70,000 to 500,000. As a result, in a case where the graft 100 is transplanted into a living body, the shell part 110 is permeable to oxygen, nutrients, substances secreted by the cells 140, and the like but is not permeable to immune cells, antibodies, and the like in the living body.

Therefore, when the graft 100 is transplanted into a living body, the cells 140 are not subjected to an immune attack of the host. Furthermore, the substances secreted by the cells 140 permeate through the shell part 110 and are secreted into the living body.

The molecular weight cut-off of the shell part 110 can be optionally controlled according to the purpose, by adjusting the density of the second hydrogel, or the like.

The cross-sectional shape of a face of the graft 100 orthogonal to the axial direction is not particularly limited and may be a circle, an elliptical system, a polygon such as a tetragon or a pentagon, or the like. Here, the axial direction of the graft may be the length direction of the graft or may be the major axis direction of a first hydrogel fiber 130.

The graft 100 has an outer diameter D. Here, the outer diameter D may be the diameter of a circle having the same area as the cross-sectional area of a face orthogonal to the axial direction of the graft. The outer diameter D may be 1 to 100 mm. The lower limit value of the outer diameter D may be 1.5 mm, may be 2 mm, may be 3 mm, may be 4 mm, may be 5 mm, or may be 6 mm. The upper limit value of the outer diameter D may be 100 mm, may be 80 mm, may be 60 mm, may be 40 mm, may be 30 mm, may be 20 mm, may be 10 mm, may be 9 mm, may be 8 mm, or may be 7 mm. The lower limit value and the upper limit value of the outer diameter D can be facultatively combined. According to an embodiment, the outer diameter D may have a size of greater than 1.5 mm.

As will be described later in the Examples, when the outer diameter D of the graft is in the above-described range, even two weeks after the graft is transplanted into a living body, the number of cells derived from the living body and adhering to the surface of the graft tends to be maintained at 5×10⁵ cells/cm² or less. Therefore, the adhesion between the tissue in the living body and the graft is suppressed, and even after a long period of time has passed since transplantation, the graft can be easily taken out of the living body as needed.

Furthermore, the length of the graft can be appropriately adjusted according to the symptom of the subject of transplantation and the purpose of transplantation, and it is preferable that the length be in the range of, for example, 1 to 100 cm.

It is preferable that the distance between the surface of the graft 100 and the cells 140 contained in the first hydrogel fibers 130 be a distance over which the cells 140 are viable. Here, the surface of the graft 100 is a face that comes into contact with a body fluid when the graft 100 is transplanted into a living body. The above-described distance is preferably 2 mm or less, and more preferably 1 mm or less.

The cells 140 contained in the first hydrogel fibers 130 may be allogeneic or xenogeneic to the living body into which the graft 110 is transplanted.

In regenerative medicine, it is often more realistic to transplant allogeneic or xenogeneic cells than to transplant autologous cells. However, when allogeneic or xenogeneic cells are transplanted into a living body, the transplanted cells are rejected by the immune system of the host, and the functions of the transplanted cells deteriorate. In contrast, according to the graft of the present embodiment, the graft is not subjected to an immune attack by the host regardless of whether the cells 140 are allogeneic or xenogeneic.

Examples of the cells 140 contained in the first hydrogel fibers 130 include endocrine cells, exocrine cells, neural cells, skeletal muscle cells, blood cells, and interstitial cells. Here, examples of the endocrine cells include neurosecretory cells, pituitary cells, thyroid gland cells, parathyroid cells, pancreatic islet cells, gastrointestinal endocrine cells, myocardial cells, liver cells, kidney cells, fat cells, adrenal cells, and gonad cells. Examples of the exocrine cells include gastrointestinal epithelial cells. Examples of the neural cells include neural stem cells, central nerve cells, peripheral nerve cells, and glial cells. Examples of the skeletal muscle cells include bone cells and cartilage cells. Examples of the blood cells include hematopoietic stem cells, white blood cells, red blood cells, and platelets. Examples of the interstitial cells include fibroblasts and vascular cells.

These cells may be cells that have been induced to differentiate from iPS cells, ES cells, or the like. As will be described later in the Examples, for example, in a case where the cells 140 are pancreatic islet cells, diabetes mellitus can be treated by transplanting the graft of the present embodiment into a living body.

The cells 140 contained in the first hydrogel fibers 130 may include a plurality of kinds of cells. As a result, cells that interact with each other can be disposed inside the graft so that the cells can be caused to interact inside the graft. The plurality of kinds of cells may be a mixture of any of the above-mentioned cells. Alternatively, the plurality of kinds of cells may be a mixture of endocrine cells, exocrine cells, neural cells, skeletal muscle cells, or blood cells with interstitial cells. Examples of the cells that interact with each other include a combination of pancreatic islet cells and vascular endothelial cells, and a combination of liver cells and non-parenchymal cells.

In a case where a plurality of kinds of cells are contained in the hydrogel fibers 130, a mixture of a plurality of kinds of cells may be contained in a single hydrogel fiber 130, or it is also acceptable that one hydrogel fiber 130 contain one kind of cells and a portion of a plurality of the first hydrogel fibers 130 contain cells of a kind different from other first hydrogel fibers 130.

### (Modification example)

In the graft 100, the disposition of the first hydrogel fibers 130 inside the core part 120 is not limited to that shown in Fig. 1(a). In Fig. 1(a), the first hydrogel fibers 130 are disposed at intervals in the peripheral part of the core part 120.

In contrast, it is also acceptable that the number of the first hydrogel fibers 130 be increased and a large number of the first hydrogel fibers 130 be disposed in close contact with the peripheral part of the core part 120. In this case, the hydrogel fibers 130 will be disposed in an annular shape. As a result, the number of the cells 140 contained in the graft 100 can be increased.

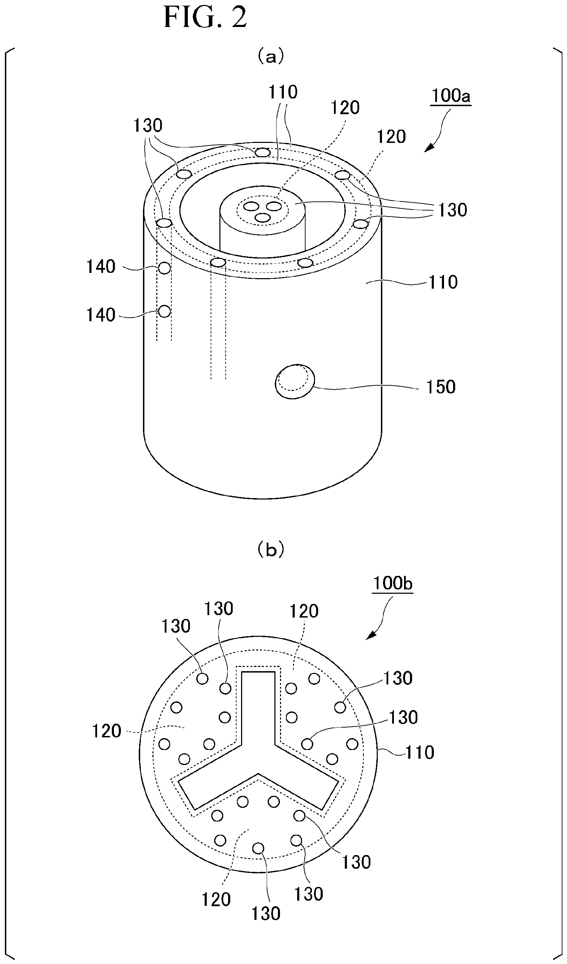

Furthermore, in the graft 100, the disposition of the shell part 110 and the core part 120 is not limited to that shown in Fig. 1(a). Fig. 2(a) is a perspective view showing the structure of a graft 100a, which is one of modification examples of the graft 100. As shown in Fig. 2(a), the graft 100a has a shell part 110 and a core part 120.

The shell part 110 of the graft 100a has a double structure of an outer side and an inner side. The core part 120 is located inside the outer shell part 110. Furthermore, the core part 120 is also located inside the inner shell part 110. The core part 120 includes a plurality of first hydrogel fibers 130. The hydrogel fibers 130 contain cells 140.

In the graft 100a, the space between the outer shell part 110 and the inner shell part 110 forms a recess into which a body fluid intrudes when the graft 100a is transplanted into a living body.

The surface of the recess is also a surface of the graft 100a. As a body fluid intrudes into the recess, the region in which the distance between the surface of the graft 100a and the cells 140 contained in the first hydrogel fibers 130 is a distance over which the cells 140 are viable can be enlarged. As a result, the cell count of the cells 140 that can survive in the graft 100a can be increased.

A through-hole 150 may be provided on the outer surface of the graft 100a. The through-hole 150 communicates the outer surface of the graft 100a with the recess of the graft 100a. In a case where the graft 100a is transplanted into a living body, a body fluid also intrudes through the through-hole 150.

Fig. 2(b) is a cross-sectional view showing the structure of a graft 100b, which is one of modification examples of the graft 100. As shown in Fig. 2(b), the graft 100b has a shell part 110 and a core part 120.

The graft 100b has a recess inside. In this example, the cross-sectional shape of the recess is such that three rectangular parallelepipeds are arranged radially from the center of the recess. When the graft 100b is transplanted into a living body, a body fluid intrudes into the recess.

In the graft 100b, the region in contact with the recess is also the shell part 110. The core part 120 is located inside the shell part 110. The core part 120 includes a plurality of first hydrogel fibers 130. The hydrogel fibers 130 contain cells 140.

### [Graft production device]

### (First embodiment)

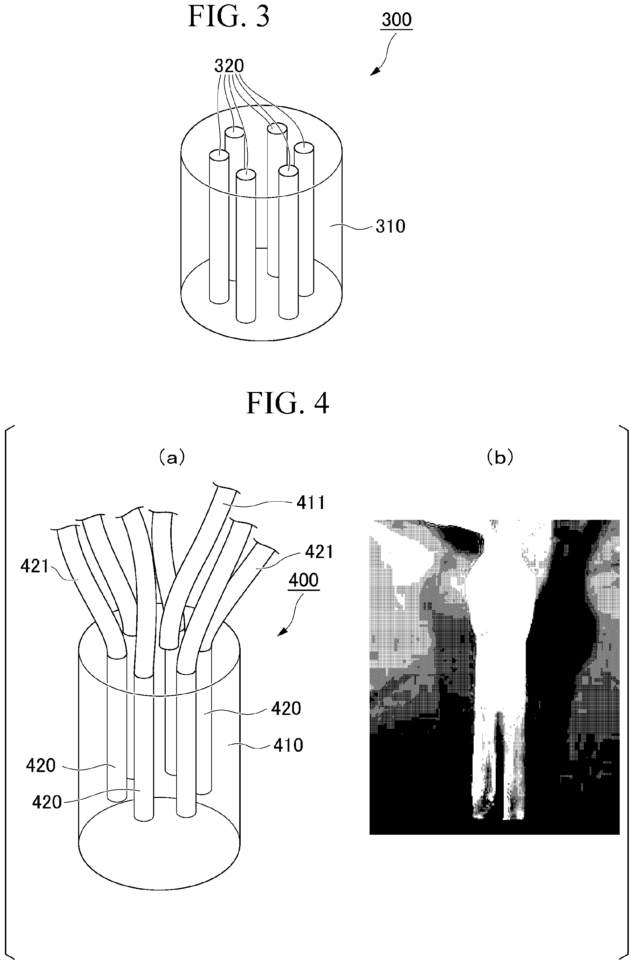

The graft production device of the first embodiment includes a container part and a plurality of columnar jigs that are attachably and detachably disposed inside the container part. Fig. 3 is a schematic diagram showing the structure of a graft production device of the present embodiment. As shown in Fig. 3, the graft production device 300 includes a container part 310 and a plurality of columnar jigs 320 that are attachably and detachably disposed inside the container part 310. The above-mentioned graft can be produced by the graft production device of the first embodiment.

### (Second embodiment)

The graft production device of the second embodiment includes a first cylindrical part and a plurality of second cylindrical parts disposed inside the first cylindrical part. Fig. 4(a) is a schematic diagram showing the structure of the graft production device of the present embodiment. As shown in Fig. 4(a), the graft production device 400 includes a first cylindrical part 410 and a plurality of second cylindrical parts 420 disposed inside the first cylindrical part 410. It is preferable that the first cylindrical part 410 be connected to a tube 411 that supplies a second sol to the first cylindrical part 410. Furthermore, it is preferable that the second cylindrical part 420 be connected to a tube 421 that supplies a first sol to the second cylindrical part 420. Fig. 4(b) is a photograph showing a specific example of a graft production device of a second embodiment. The above-mentioned graft can be produced by the graft production device of the second embodiment.

### [Method for producing graft]

### (First embodiment)

A method for producing a graft according to the first embodiment is a production method using the above-described graft production device according to the first embodiment. In the method of the present embodiment, first, a second sol is injected into the container part 310 of the graft production device 300, and a second hydrogel is formed.

Subsequently, after the formation of the second hydrogel, the plurality of columnar jigs 320 are removed. As a result, a second hydrogel is obtained in which the portions where the columnar jigs 320 have existed form recesses. Subsequently, a first sol containing cells is injected into the recesses of the second hydrogel, and a first hydrogel is formed. Regarding the cells, those described above can be used. As a result, the above-mentioned graft can be produced.

It is preferable that the method for producing a graft according to the first embodiment further include, after forming the first hydrogel, a step of covering an exposed portion of the first hydrogel with the second hydrogel. Thereby, the first hydrogel containing cells can be completely covered with the second hydrogel. As a result, the cells are completely encapsulated in the second hydrogel, and when the graft is transplanted into a living body, the possibility of the cells being subjected to an immune attack can be further lowered.

In the production method of the present embodiment, as the sol, a sol that forms a hydrogel by gelling in the presence of metal ions, a sol that gels in response to temperature and forms a hydrogel, a sol that gels in response to pH and forms a hydrogel, a sol that forms a hydrogel in response to light, a sol that forms a hydrogel in response to a magnetic field, or the like can be used. Hereinafter, a sol and a gel may be referred to without distinguishing them.

In order to gel the sol, operations such as bringing metal ions into contact with the sol, adjusting the temperature to the gelling conditions, adjusting the pH to the gelling conditions, irradiating light under the gelling conditions, and applying a magnetic field under the gelling conditions, may be performed according to the characteristics of the gel used.

Examples of a hydrogel that gels in the presence of metal ions include an alginate gel that gels in the presence of divalent or trivalent metal ions, a carrageenan gel that gels in the presence of calcium ions and potassium ions, and an acrylic acid-based synthetic gel that gels in the presence of sodium ions.

Examples of the temperature-responsive hydrogel include a temperature-responsive hydrogel obtained by cross-linking poly(N-isopropylacrylamide) with polyethylene glycol (commercial name: MEBIOL GEL), a copolymer of methyl cellulose, hydroxypropyl cellulose, or polylactic acid and polyethylene glycol, a triblock copolymer of polyethylene glycol and polypropylene oxide (commercial name: PLURONIC, POLOXAMER), agarose, and polyvinyl alcohol.

Examples of the pH-responsive hydrogel include an alginate gel, a chitosan gel, a carboxymethyl cellulose gel, and an acrylic acid-based synthetic gel.

Examples of the photoresponsive hydrogel include a synthetic gel in which azobenzene and cyclodextrin are combined in the skeleton, a gel formed from a supermolecule having fumaric acid amide as a spacer, and a gel cross-linked or bonded through a nitrobenzyl group.

Examples of the magnetic field-responsive hydrogel include a gel formed from cross-linked poly(N-isopropylacrylamide) containing magnetic particles.

### (Second embodiment)

A method for producing a graft according to the second embodiment is a production method of using the graft production device according to the second embodiment described above. In the method of the present embodiment, a graft is produced by forming a laminar flow of a first sol and a laminar flow of a second sol and gelling them. It is preferable that either the first sol or the second sol gel earlier than the other.

Specifically, a step of supplying a second sol through one side of the graft production device 400 (upper side in Fig. 4(a)) to the first cylindrical part 410, and forming a laminar flow of the second sol that is discharged through the other side of the graft production device 400 (lower side in Fig. 4(a)) while gelling the second sol to form a second hydrogel; and a step of supplying a first sol containing cells through one side of the graft production device 400 (upper side in Fig. 4(a)) to the second cylindrical part 420, and forming a laminar flow of the first sol that is discharged through the other side of the graft production device 400 (lower side in Fig. 4(a)) while gelling the first sol to form a first hydrogel are carried out simultaneously. Regarding the cells, those described above can be used. As a result, the above-mentioned graft can be produced.

With regard to the method for producing a graft according to the second embodiment, it is preferable that a step of forming only the second hydrogel be further included before and after the step of synchronously carrying out the step of forming the second hydrogel and the step of forming the first hydrogel.

That is, it is preferable that first a laminar flow of only a second sol be formed while the second sol is gelled to form a second hydrogel, subsequently a step of forming a second hydrogel and a step of forming a first hydrogel be carried out synchronously as described above, and subsequently, a laminar flow of only a second sol be formed while the second sol is gelled to form a second hydrogel.

As a result, in the graft thus produced, the first hydrogel containing cells can be completely covered with the second hydrogel. As a result, the cells are completely encapsulated in the second hydrogel, and when the graft is transplanted into a living body, the possibility of the cells being subjected to an immune attack can be further lowered.

The supply of the second sol to the first cylindrical part 410 can be performed through a tube 411. Furthermore, the supply of the first sol to the second cylindrical part 420 can be performed through a tube 421.

With regard to the production method of the second embodiment, the same sols and hydrogels as those used in the production method of the first embodiment can be used.

### [Other embodiments]

According to an embodiment, the present invention provides a therapeutic method for a disease, the therapeutic method including transplanting a graft containing an effective number of cells into a patient in need of treatment, in which the graft has a shell part that comes into contact with a body fluid in a case where the graft is transplanted into a living body; and a core part that is located inside the shell part and does not substantially come into contact with the body fluid, the core part contains a plurality of first hydrogel fibers containing the cells, the shell part contains a second hydrogel functioning as a semipermeable membrane, and the graft has a size with an outer diameter of greater than 1.5 mm.

In the therapeutic method of the present embodiment, the type of cells is selected according to the disease. For example, it is effective to transplant pancreatic islet cells for the treatment of diabetes mellitus. Furthermore, it is effective to transplant liver cells for the treatment of a liver disease. In addition, it is effective to transplant nerve cells for the treatment of Parkinson's disease.

### Examples

Hereinafter, the present invention will be described by way of Experimental Examples; however, the present invention is not intended to be limited to the following Experimental Examples.

### [Experimental Example 1]

### (Production of hydrogel fiber)

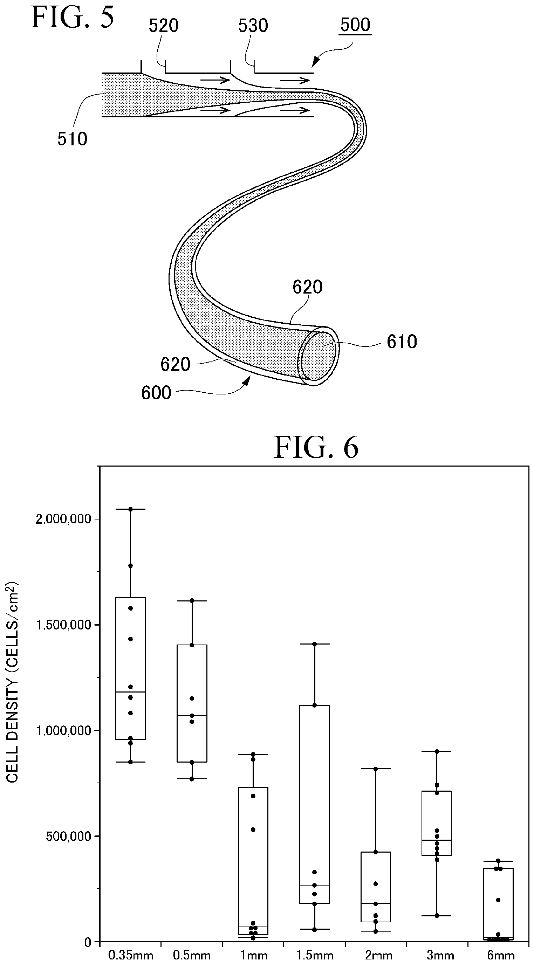

First, a method for producing a hydrogel fiber will be described. The method for producing a hydrogel fiber is not particularly limited; however, a fiber can be conveniently produced by using, for example, a double coaxial microfluidic device as shown in Fig. 5. For example, a microfluidic device that can separately inject two fluids into a core part and a shell part so as to be coaxial is specifically described also in Fig. 1 of Wonje Jeong, et al., Hydrodynamic microfabrication via "on the fly" photopolymerization of microscale fibers and tubes, Lab Chip, 2004, 4, 576-580.

Fig. 5 is a schematic diagram showing an example of the production process for a hydrogel fiber 600. Here, a case where a collagen solution is used as the material for the core part and a sodium alginate solution prior to cross-linking is used as the material for the shell part will be described.

First, a collagen solution is introduced and injected through an inlet port 510 of a microfluidic device 500. Furthermore, a sodium alginate solution prior to cross-linking is introduced and injected through an inlet port 520 of the microfluidic device 500. In addition, a calcium chloride solution is introduced and injected through an inlet port 530 of the microfluidic device 500. Then, the sodium alginate solution in the shell part gels, and a hydrogel fiber in which the core part 610 is a collagen gel and the shell part 620 is an alginate gel can be produced.

The injection speed for the solutions at the inlet ports 510 and 520 is not particularly limited; however, in a case where the port diameter of the microfluidic device 500 is about 50 µm to 2 mm, the injection speed may be about 10 to 500 µL/min. By regulating the injection speed for the solutions at the inlet ports 510 and 520, the diameter of the core part and the coating thickness of the shell part can be appropriately regulated. The injection speed for the solution at the inlet port 530 is not particularly limited; however, the injection speed may be, for example, about 1 to 10 mL/min.

In the present Experimental Example, hydrogel fibers having different outer diameter sizes were produced using the microfluidic device shown in Fig. 5. The outer diameter sizes were set to 0.35 mm, 0.5 mm, 1 mm, 1.5 mm, 2 mm, 3 mm, and 6 mm. Furthermore, the length of the hydrogel fibers was set to 3 cm.

As shown in Fig. 5, a collagen solution or a laminin solution including alginic acid was supplied as a core fluid to a coaxial microchannel of the microfluidic device. Furthermore, an aqueous solution of sodium alginate was supplied as a shell fluid. In addition, an aqueous solution of barium chloride was supplied as a sheath fluid. As a result, gelation was induced at the interface between the shell and the sheath, and a hydrogel fiber was obtained. The shell of the hydrogel fiber was a gel of barium alginate.

### [Experimental Example 2]

### (Transplantation of hydrogel)

The various hydrogel fibers produced in Experimental Example 1 were each transplanted into the abdominal cavity of a healthy C57BL/6 mouse having an immune function, and the effects of the fibers were examined.

After 14 days had passed from the transplantation of the various hydrogel fibers, the various hydrogel fibers were taken out from the abdominal cavities of the mice. The period of 14 days is considered to be a period suitable for observing a Foreign Body Reaction (FBR) against a transplanted tissue.

Subsequently, the number of cells adhering to the surface of each hydrogel fiber was counted. Fig. 6 is a graph showing the results of measuring the numbers of cells adhering to the surface of various hydrogel fibers. The axis of ordinate of the graph represents the cell density (cells/cm²).

As a result, it was made clear that cell adhesion to hydrogel fibers having an outer diameter of 0.35 mm and 0.5 mm occurred to a large extent; however, cell adhesion to hydrogel fibers having an outer diameter of 1 mm or greater occurred to a lesser extent. These results indicate that when the outer diameter of the graft is 1 mm or greater, the adhesion between the tissue in the living body and the graft is suppressed.

### [Experimental Example 3]

### (Production of graft)

A graft was produced using the graft production device shown in Figs. 4(a) and 4(b). A collagen solution or a laminin solution was used as a first sol. The first sol was a sol that gels under temperature conditions at a certain temperature or higher. In addition, human pancreatic islet cells induced to differentiate from human iPS were mixed into the first sol. An alginic acid solution was used as a second sol. The second sol was a sol that gels when it reacts with a liquid including a multivalent cation.

A step of supplying a second sol through one side of a graft production device to a first cylindrical part, and forming a laminar flow of the second sol that is discharged through the other side of the graft production device while gelling the second sol to form a second hydrogel; and a step of supplying a first sol containing cells through the one side of the graft production device to a second cylindrical part, and forming a laminar flow of the first sol that is discharged through the other side of the graft production device while gelling the first sol to form a first hydrogel were carried out simultaneously. As a result, a graft was formed at the other side of the graft production device.

The graft had a cylindrical shape with a length of 20 to 30 mm and an outer diameter of 6 mm. Furthermore, two kinds of grafts containing 6×10⁶ cells or 1.2×10⁷ cells of pancreatic islet cells per graft were produced.

### [Experimental Example 4]

### (Transplantation of graft into model mouse of diabetic immunodeficiency)

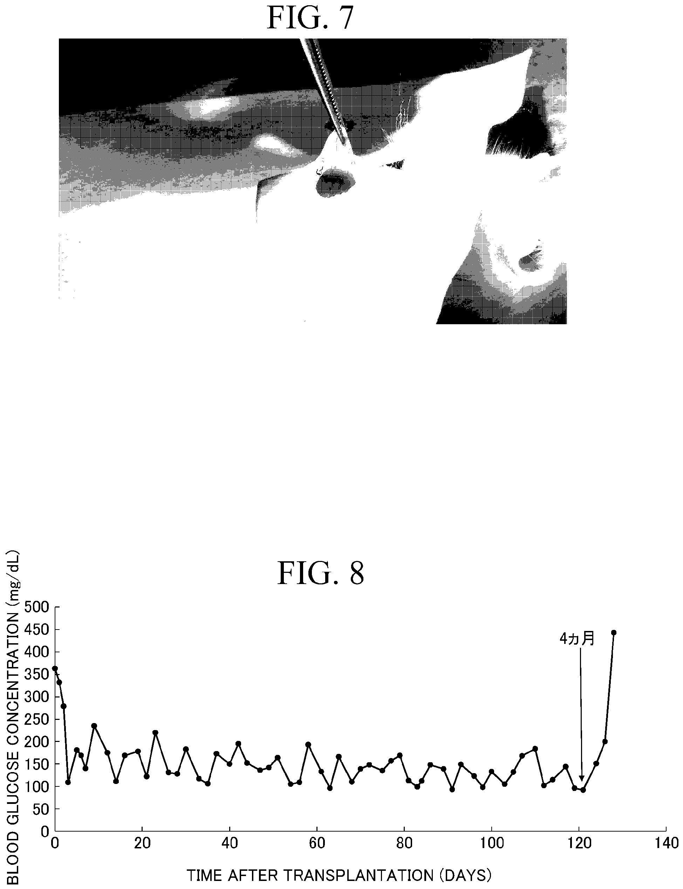

The graft produced in Experimental Example 3 was transplanted intraperitoneally or subcutaneously into a model mouse of diabetic immunodeficiency, and the transition of the blood glucose concentration was measured. As the graft, grafts containing 1.2×10⁷ pancreatic islet cells per graft were used. Furthermore, NOD-Scid mice were used as the model mice of diabetic immunodeficiency. Fig. 7 is a photograph showing a state in which the graft is being transplanted into a mouse.

The non-fasting blood glucose concentrations of the mice after having the grafts transplanted were measured at least once a day. A blood glucose concentration of 200 mg/dL or less was considered to be normoglycemia. Fig. 8 is a graph showing the results of measuring the transition in the blood glucose concentration in the mice.

As a result, it was made clear that the blood glucose concentration, which was 200 mg/dL or greater before the transplantation of a graft, was maintained at 200 mg/dL or less after the transplantation of the graft. Moreover, when the graft was retrieved 4 months after the transplantation, the blood glucose concentration increased to 200 mg/dL or greater again. This result indicates that the blood glucose concentration was maintained to be 200 mg/dL or less due to the presence of the graft.

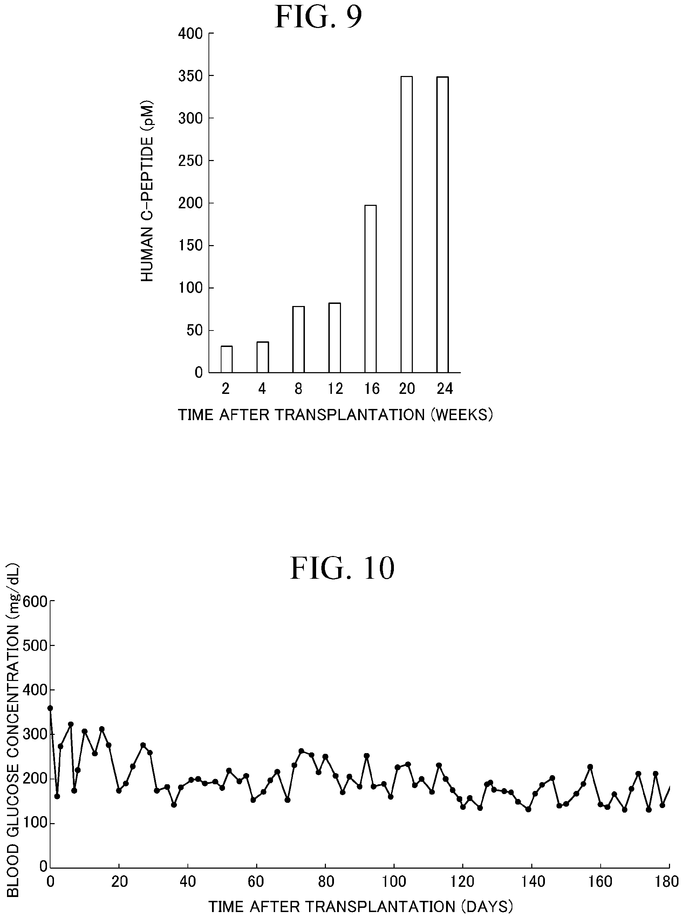

Furthermore, the concentration of human C-peptide in the blood of mice was measured. Fig. 9 is a graph showing the results of measuring the concentration of human C-peptide. C-peptide is a constituent component of proinsulin, which is a precursor of insulin. Proinsulin is separated into an insulin moiety and a C-peptide moiety having 31 amino acids in the pancreatic islet cells, and these moieties are released into the blood. Therefore, it can be said that the concentration of human C-peptide in the blood corresponds to the amount of human insulin secreted by human pancreatic islet cells contained in the graft. Human C-peptide can be measured even in the presence of anti-human insulin antibody and mouse insulin.

From the results of Fig. 9, it was verified that the functions of human pancreatic islet cells contained in the graft were maintained even 24 weeks after the transplantation of the graft.

### [Experimental Example 5]

### (Transplantation of graft into diabetic model mouse having immune function)

The graft produced in Experimental Example 3 was transplanted intraperitoneally or subcutaneously into a diabetic model mouse having an immune function, and the transition of the blood glucose concentration was measured. As the graft, grafts containing 6×10⁶ pancreatic islet cells per graft were used. Furthermore, as the diabetic mouse having an immune function, C57BL/6 mice in which diabetes mellitus was induced by administering streptozocin (CAS number: 18883-66-4) were used.

The non-fasting blood glucose concentrations of the mice after having the grafts transplanted were measured at least once a day. A blood glucose concentration of 200 mg/dL or less was considered to be normoglycemia. Fig. 10 is a graph showing the results of measuring the transition in the blood glucose concentration in the mice.

As a result, a tendency for the blood glucose concentration, which was 200 mg/dL or greater before the transplantation of the graft, to be maintained at 200 mg/dL or less after the transplantation of the graft was recognized. This effect was maintained for at least 180 days after transplantation.

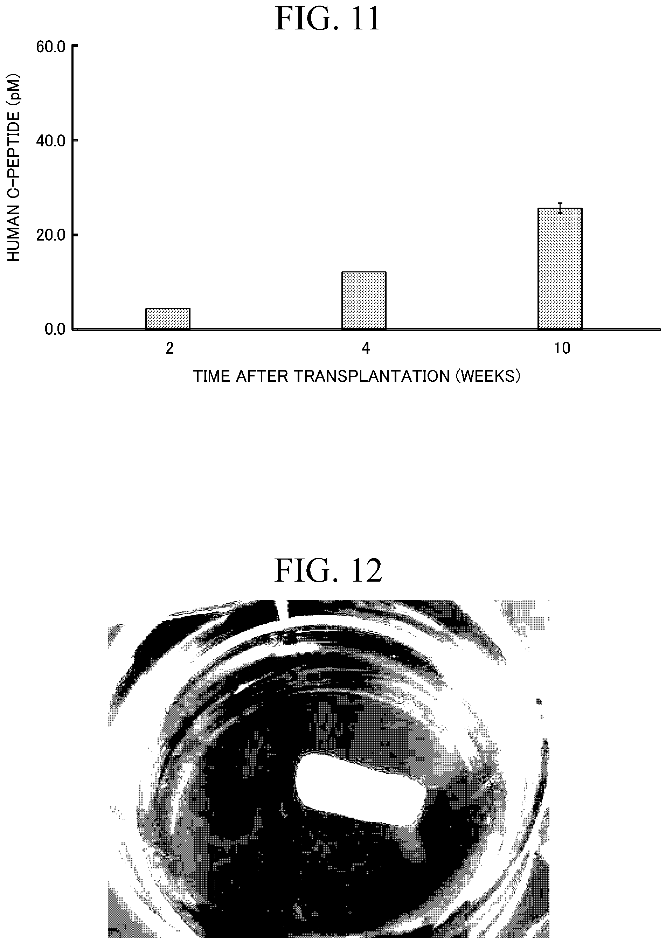

Furthermore, the concentration of human C-peptide in the blood of mice was measured. Fig. 11 is a graph showing the results of measuring the concentration of human C-peptide in the blood of mice. From the results shown in Fig. 11, it was verified that the function of human pancreatic islet cells contained in the graft was maintained even 10 weeks after the transplantation of the graft.

### [Experimental Example 6]

### (Examination of graft)

From the C57BL/6 mice having the grafts transplanted in Experimental Example 5, the grafts were extracted and analyzed after 4 months. Fig. 12 is a photograph of an extracted graft.

First, the grafts were incubated in a medium, the glucose concentration in the medium was adjusted to 3 mg/mL, and the concentrations of human C-peptide and glucagon secreted into the medium when a hypoglycemic state was attained were measured. Subsequently, the glucose concentration in the medium was adjusted to 22 mg/mL, and the concentrations of human C-peptide and glucagon secreted into the medium when a hyper-hypoglycemic state was attained were measured. Subsequently, the glucose concentration in the medium was adjusted to 3 mg/mL, and the concentrations of human C-peptide and glucagon secreted into the medium when a hypoglycemic state was attained again were measured. Glucagon is a peptide hormone secreted from pancreatic islet cells when the blood glucose level drops and sugar is needed, as opposed to insulin.

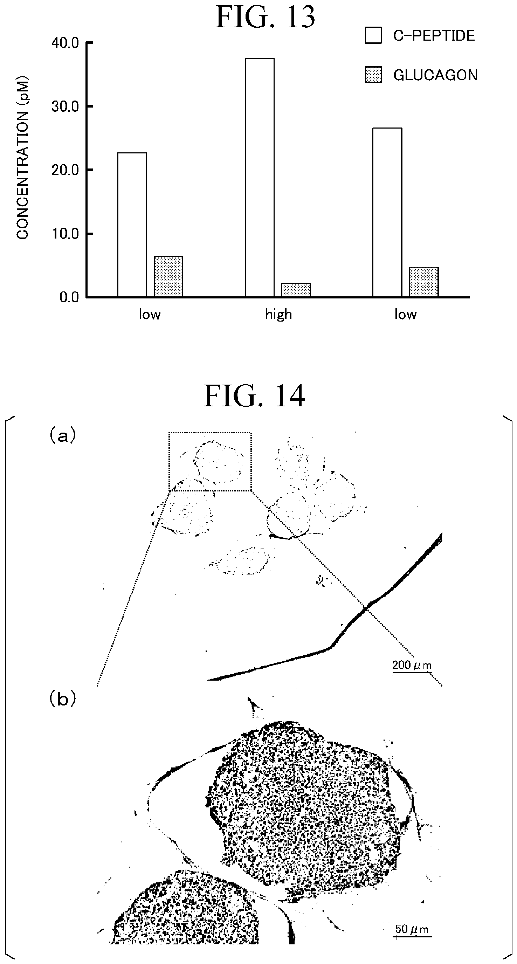

Fig. 13 is a graph showing the results of measuring the concentrations of C-peptide and glucagon. In Fig. 13, the term "low" indicates the measurement results obtained in a hypoglycemic state, and the term "high" indicates the measurement results obtained in a hyperglycemic state.

Subsequently, thin sliced sections of the extracted graft were produced and stained with hematoxylin and eosin. Figs. 14(a) and 14(b) are microphotographs showing the results of hematoxylin and eosin staining. Fig. 14(b) is a microphotograph taken by magnifying a portion of Fig. 14(a).

As a result, it was verified that the human pancreatic islet cells contained in the graft were alive even though the graft had been transplanted into the body of the mouse for 4 months.

In addition, a similar analysis was performed after 4 months by extracting the graft also from the NOD-Scid mouse into which a graft had been transplanted in Experimental Example 4. As a result, results similar to those of the present Experimental Example were obtained. That is, it was verified that also in the NOD-Scid mouse, the human pancreatic islet cells contained in the graft were still alive, even though the graft had been transplanted into the body of the mouse for 4 months. Furthermore, it was verified that the human pancreatic islet cells contained in the extracted graft changed the concentrations of C-peptide and glucagon according to the glucose concentration in the medium.

### Industrial Applicability

According to the present invention, a graft can be provided with which cells can be transplanted into a living body without being subjected to an immune attack and can also be taken out of the living body.

### Reference Signs List

- 100, 100a, 100b:: Graft
- 110, 620:: Shell part
- 120, 610:: Core part
- 130:: First Hydrogel fiber
- 140:: Cell
- 150:: Through-hole
- 300, 400:: Graft Production device
- 310:: Container part
- 320:: Columnar jig
- 410:: First cylindrical part
- 420:: Second cylindrical part
- 411,421:: Tube
- 500:: Microfluidic device
- 510, 520, 530:: Inlet port
- 600:: Hydrogel fiber
- D:: Outer diameter

## Claims

1. A graft comprising:
a shell part that comes into contact with a body fluid when the graft is transplanted into a living body; and a core part that is located inside the shell part and does not substantially come into contact with the body fluid,
wherein the core part contains a plurality of first hydrogel fibers containing cells,
the shell part contains a second hydrogel that functions as a semipermeable membrane, and
the graft has a size with an outer diameter of 1 mm or greater.

2. The graft according to Claim 1, wherein the semipermeable membrane has a molecular weight cut-off of 70,000 to 500,000.

3. The graft according to Claim 1 or 2, wherein the graft has a recess into which the body fluid intrudes when the graft is transplanted into the living body.

4. The graft according to any one of Claims 1 to 3, wherein two weeks after being transplanted into the living body, the number of cells derived from the living body and adhering to a surface is 5×10⁵ cells/cm² or less.

5. The graft according to any one of Claims 1 to 4, wherein the cells contained in the first hydrogel fibers are allogeneic or xenogeneic to the living body.

6. The graft according to any one of Claims 1 to 5, wherein a distance between a surface of the graft and the cells contained in the first hydrogel fibers is a distance over which the cells are viable.

7. The graft according to any one of Claims 1 to 6, wherein the cells contained in the first hydrogel fibers are cells of at least one selected from the group consisting of endocrine cells, exocrine cells, neural cells, skeletal muscle cells, blood cells, and interstitial cells.

8. The graft according to any one of Claims 1 to 7, wherein the cells contained in the first hydrogel fibers include a plurality of kinds of cells.

9. A graft production device, comprising:
a container part; and
a plurality of columnar jigs that are attachably and detachably disposed inside the container part.

10. A method for producing the graft according to any one of Claims 1 to 8, the method comprising:
a step of injecting a second sol into the container part of the graft production device according to Claim 9 and forming a second hydrogel;
a step of removing the plurality of columnar jigs after the formation of the second hydrogel and obtaining a second hydrogel in which the portions where the columnar jigs have existed form recesses; and.
a step of injecting a first sol containing cells into the recesses and forming a first hydrogel.

11. A graft production device, comprising:
a first cylindrical part; and
a plurality of second cylindrical parts disposed inside the first cylindrical part.

12. A method for producing the graft according to any one of Claims 1 to 8, the method comprising:
a step of supplying a second sol through one side of the graft production device according to Claim 11 into the first cylindrical part, and forming a laminar flow of the second sol that is discharged through the other side of the graft production device while gelling the second sol to form a second hydrogel; and
a step of supplying a first sol containing cells through one side of the graft production device into the second cylindrical parts, and forming a laminar flow of the first sol that is discharged through the other side of the graft production device while gelling the first sol to form a first hydrogel, the two steps being carried out simultaneously.
